# EUROPEAN PATENT SPECIFICATION

(11) **EP 3 885 433 B1**
(45) Date of publication and mention of the grant of the patent: **02.07.2025**
(21) Application number: 19887734.2
(22) Date of filing: 22.11.2019
(51) Int. Cl.: C12N 5/077, A61K 35/28, A23L 33/10, A61P 3/00, A61P 3/04, A61P 3/06

(54) **COMPOSITION, COMPRISING MESENCHYMAL STEM CELLS, FOR INHIBITING ADIPOGENESIS**
ZUSAMMENSETZUNG MIT MESENCHYMALEN STAMMZELLEN ZUR HEMMUNG DER ADIPOGENESE
COMPOSITION, COMPRENANT DES CELLULES SOUCHES MÉSENCHYMATEUSES, POUR INHIBER L'ADIPOGENÈSE

(30) Priority: 23.11.2018 KR 20180146782
(43) Date of publication of application: 29.09.2021
(73) Proprietor: Cha University Industry-Academic Cooperation Foundation, Pocheon-si, Gyeonggi-do 11160 (KR); Sungkwang Medical Foundation, Seoul 06135 (KR)
(72) Inventor: KIM, Gi Jin, Seoul 06084 (KR); KIM, Jae Yeon, Seoul 06078 (KR); LEW, He Len, Seoul 06279 (KR)
(74) Representative: Weickmann & Weickmann PartmbB
(86) International application number: PCT/KR2019/016123
(87) International publication number: WO 2020/106097

(56) References cited:
- WO-A1-2004/050117
- KR-A- 20170 022 369
- IRINA ARUTYUNYAN ET AL: "Umbilical Cord as Prospective Source for Mesenchymal Stem Cell-Based Therapy", STEM CELLS INTERNATIONAL, vol. 2016, 1 January 2016 (2016-01-01), US, pages 1 - 17, XP055681679, ISSN: 1687-966X, DOI: 10.1155/2016/6901286
- KOBAYASHI MICHIHIRO ET AL: "PRL2/PTP4A2 Phosphatase Is Important for Hematopoietic Stem Cell Self-Renewal", STEM CELLS, vol. 32, no. 7, 1 July 2014 (2014-07-01), pages 1956 - 1967, XP055935664, ISSN: 1066-5099, Retrieved from the Internet <URL:https://www.ncbi.nlm.nih.gov/pmc/articles/PMC4063874/pdf/nihms-566444.pdf> DOI: 10.1002/stem.1672
- JONG HO CHOI , GI DAE KIM , JIN SEOK ,SI HYUN BAE ,SOON KOO BAIK , HOON OH, GI JIN KIM : "PO-004: Increased phosphatase of regenerating-1 by placental stem cells promote hepatic regeneration in a rat model with bile duct ligation", SPRING/AUTUMN ACADEMIC CONFERENCE KASL, vol. 2016, no. 1, 30 November 2015 (2015-11-30), Korea, pages 82 - 82, XP009528036
- HWANG, BYUNGTAE: "The Multifunctional Roles of PTP4A1 in Angiogenesis and Insulin Resistance", PHD THESIS, August 2017 (2017-08-01), KR, pages 1 - 97, XP009532636
- P. FLACHS ; O. HORAKOVA ; P. BRAUNER ; M. ROSSMEISL ; P. PECINA ; N. FRANSSEN-VAN HAL ; J. RUZICKOVA ; J. SPONAROVA ; Z. DRAHOTA ;: "Polyunsaturated fatty acids of marine origin upregulate mitochondrial biogenesis and induce 13-oxidation in white fat", DIABETOLOGIA, vol. 48, no. 11, 5 October 2005 (2005-10-05), pages 2365 - 2375, XP019322371, ISSN: 1432-0428, DOI: 10.1007/s00125-005-1944-7
- S-H MIN, KIM D M, HEO Y-S, KIM Y-I, KIM H M, KIM J, HAN Y-M, KIM I-C, YOO O-J: "New p53 target, phosphatase of regenerating liver 1 (PRL-1) downregulates p53", ONCOGENE, vol. 28, no. 4, 10 November 2008 (2008-11-10), pages 545 - 554, XP055710507, ISSN: 0950-9232, DOI: 10.1038/onc.2008.409
- KIM, J. Y.: "Dynamic regulation of miRNA expression by functionally enhanced placental mesenchymal stem cells promotes hepatic regeneration in a rat model with bile duct ligation", INTERNATIONAL JOURNAL OF MOLECULAR SCIENCES, vol. 20, no. 5299, 24 October 2019 (2019-10-24), pages 1 - 18, XP055710513, DOI: 10.3390/ijms20215299
- GI JIN KIM: "Feasibilityof enhanced PRL-1 in placenta-derived mesenchymal stem cells by gene modification on a rat model with hepatic failure", 20 June 2019 (2019-06-20), pages 1 - 1, XP055710515, Retrieved from the Internet <URL:https://www.kasl.org/pdf/2019_liver/8_2.pdf>

## Description

### TECHNICAL FIELD

The present disclosure relates to a composition for use in inhibiting, preventing, alleviating, or treating adipogenesis. The present application claims the benefit of Korean Patent Application No. 10-2018-01467892, filed on November 23, 2018, in the Korean Intellectual Property Office.

### BACKGROUND ART

Adipogenesis and fat accumulation are caused by metabolic syndrome or autoimmune responses and cause excessive adipocyte metabolism, resulting in an increase in adipose tissues. Treatment methods aimed at alleviating the symptom of fat accumulation in various diseases have included dietary therapy or drug therapy. However, to date there has been no method developed that can fundamentally treat fat accumulation.

Therefore, there is a need to develop novel therapeutic agents that are effective as well as safe, for fundamentally treating adipogenesis and fat accumulation.

### DESCRIPTION OF EMBODIMENTS

### TECHNICAL PROBLEM

The subject-matter of the invention is defined by the appended claims. Any other aspects described herein are only provided to illustrate the background of the invention. One aspect provides a mesenchymal stem cell overexpressing phosphatase of regenerating liver-1 (PRL-1) compared to non-genetically engineered parent cell.

Another aspect provides a pharmaceutical composition for use in preventing or treating a disease associated with abnormal adipogenesis or abnormal fat metabolic dysfunction, the pharmaceutical composition comprising a mesenchymal stem cell overexpressing PRL-1 compared to a non-genetically engineered parent cell.

Another aspect described herein but not claimed is a health functional food for preventing or treating a disease associated with abnormal adipogenesis or abnormal fat metabolic dysfunction, the health functional food including a mesenchymal stem cell secreting PRL-1 or overexpressing PRL-1 compared to a parent cell.

Another aspect provides a method of inhibiting fat accumulation, the method comprising: contacting the mesenchymal stem cell with a cell in vitro, the mesenchymal stem cell overexpressing PRL-1 compared to a non-genetically engineered parent cell.

### SOLUTION TO PROBLEM

According to one aspect defined in claims 1-3, provided is a mesenchymal stem cell overexpressing PRL-1 compared to a non-genetically engineered parent cell.

The term "mesenchymal stem cell (MSC)" used in the present specification may refer to cells that maintain the ability to self-renew and stemness and are capable of being differentiated into various mesenchymal tissues, and may include mesenchymal stem cells of an animal, such as mammals, including humans. Further, MSCs may be umbilical cord-derived MSCs, umbilical cord blood-derived MSCs, bone marrow-derived MSCs, placenta-derived MSCs, or adipose-derived MSCs. The placenta-derived MSCs may be derived from various tissues constituting the placenta, for example, tissues in such as amniotic epithelial cells, amnion, trophoblast, and chorion. Preferably, the placenta-derived MSCs may be MSCs derived from the chorionic plate of the placenta, and more preferably, may be MSCs derived from the chorionic plate membrane. Isolation of the MSCs may be performed by methods obvious to those skilled in the art, and examples of such methods are described in literature by Pittenger et al. (Science 284: 143, 1997) and Van et al. (J. Clin. Invest., 58: 699, 1976). The MSCs may be ones engineered to overexpress PRL-1 or an active fragment thereof.

The MSCs may have the following characteristics a) or b):
a) the characteristics of expressing PRL-1; or
b) the characteristics of one or more cell surface antigens selected from CD34, CD105, HLA-ABC, and HLA-G.

The MSCs may be ones expressing CD34, CD105, HLA-ABC, or HLA-G. In detail, in terms of cell markers expressed on the cell surface, the MSCs provided in the present specification may be ones expressing CD34, CD105, HLA-ABC, or HLA-G positive cell surface marker in an amount of at least 20%, 25%, 30%, 35%, 40%, 45%, 50%, 55%, 60%, 65%, 70%, 75%, 80%, 85%, 90%, 95%, 98% or about 99%. The term "positive" used in the present disclosure with regard to a stem cell marker, may mean that a marker exists in a greater amount or in a higher concentration compared to other non-stem cells about the same marker. That is, because a marker exists inside a cell or on the surface thereof, if the cell can be distinguished from other types of cells by using said marker, the cell is considered to be positive for said marker. Further, it may also mean that the cell possesses said marker in an amount that can produce a signal of a greater magnitude than a background value, for example, a signal from a cell measurement device. For example, a cell can be marked so as to be detectable by a CD90-specific antibody, and if a signal from this antibody is detectably greater than a control group (for example, a background value), then the cell is considered to be "CD90+". Likewise, the term "negative" used in the present specification refers to a case in which even when an antibody specific to a particular cell surface marker is used, the marker is undetectable compared to a background value. For example, when a cell cannot be marked to be detectable with a CD45-specific antibody, the cell is considered to be "CD45-".

For the MSCs, culture, lysates, or extracts thereof may be used alternatively. The culture, lysates, or extracts may serve as a useful alternative when it is difficult to use cells as is, and because such culture, lysates, or extracts contain constitutive components of the cells, including proteins, etc., they may exhibit biological activity analogous or equivalent to that of their original cells. The lysates or extracts may be obtained using a commercially available cell lysis kit, extraction kit, or the like.

The term "phosphatase of regeneration liver-1 (PRL-1)" used in the present specification may include PRL-1 derived from vertebrate animals including a human, for example, mammals, fish, amphibians, birds, or reptiles. In addition, the PRL-1 may mean to include precursors of PRL-1.

In addition, the MSCs may be MSCs that are engineered, by a culture environment or genetically, to overexpress PRL-1 compared to a non-genetically engineered parent cell.

The term "culture environment" used in the present specification may mean the same as "culture conditions", and the term "engineering by a culture environment" and cells "engineered by a culture environment" used in the present specification refer to an act of treating cells being cultured with a particular compound, and the cells thus created, respectively. The term "genetic engineering" and cells "genetically engineered" refer to an act of introducing one or more genetic modifications into a cell, and the cells thus created, respectively. For example, the MSCs or host cells may be ones that are genetically engineered to increase the expression or activity of PRL-1 or an active fragment thereof, for example, ones including an exogeneous gene that encodes PRL-1 or an active fragment thereof. The above activity increase may mean, compared to the activity of an endogenous protein or enzyme that a given parent cell that is not genetically engineered (i.e., wild type) does or does not have, the activity of the same type of protein or enzyme having a higher activity. The exogenous gene may be expressed in a sufficient amount to induce an increase in activity of the aforementioned protein in the MCSs or host cells, compared to its parent cells. The exogenous gene may be one introduced into the parent cell through an expression vector. In addition, the exogenous gene may be one introduced into the parent cell in the form of a linear polynucleotide. In addition, the exogenous gene may be one expressed from an expression vector (i.e., a plasmid) inside cells. In addition, the exogenous gene may be, for a reliable expression, inserted in a genetic material (i.e., a chromosome) inside the cell to be expressed. In detail, the genetic engineering may be performed by a transformation technique, a transfection technique, and a transduction method, virus infection, a gene gun or Ti-mediated gene transfer technique, and more specifically, may be performed by a method selected from microinjection, electroporation, a DEAE-dextran treatment transfection method, lipofection, a nanoparticle-mediated transfection method, a protein transduction domain-mediated transfection method, a calcium phosphate (CaPO₄) transfection method, a virus-mediated gene transfer method, and a PEG-mediated transfection method. In a specific example, the genetic engineering may be performed by electroporation, but is not limited thereto.

The PRL-1 or an active fragment thereof may be prepared as a fusion protein. In a method of preparing the fusion protein, a polynucleotide encoding the PRL-1 or an active fragment thereof may be ligated to a frame with polynucleotides encoding other proteins or peptides, and the frame may be inserted into an expression vector for expression in a host. Techniques known in the art may be used for the above purpose. For peptides fused to PRL-1, known peptides may be used, such as FLAG (Hopp, T. P. et al., BioTechnology (1988) 6, 1204-1210), 6x histidine (His) residues consisting of six His, 10x His, influenza hemagglutinin (HA), human c-myc fragments, VSV-GP fragments, p18HIV fragments, T7-tag, HSV-tag, E-tag, SV40T antigen fragments, 1ck tag, alpha-tubulin fragments, B-tag, and protein C fragments. In addition, to prepare the fusion protein, the PRL-1 or an active fragment thereof may be ligated to glutathione-S-transferase (GST), influenza hemagglutinin(HA), immunoglobulin constant regions, beta-galactosidase, maltose-binding protein (MBP), or the like.

Accordingly, in other specific examples, a polynucleotide encoding the PRL-1 or an active fragment thereof, a recombinant vector including the polynucleotide, and a recombinant cell including the recombinant vector may be provided.

The term "vector" as used herein refers to a means for expressing a target gene in a host cell. For example, the vector includes plasmid vectors, cosmid vectors, and viral vectors such as bacteriophage vectors, adenovirus vectors, retrovirus vectors, and adeno-associated virus vectors. Vectors that can be used for the above recombinant vector may be prepared by altering plasmids (e.g., pSC101, pGV1106, pACYC177, ColE1, pKT230, pME290, pBR322, pUC8/9, pUC6, pBD9, pHC79, plJ61, pLAFR1, pHV14, pGEX series, pET series, pUC19, etc.), phages, or viruses (e.g., SV40, etc.) that are often used in the art.

A polynucleotide encoding the above protein complex in the above recombinant vector may be operatively linked to a promoter. As used herein, the term "operatively linked" refers to functional linkage between a nucleotide expression regulatory sequence (e.g., a promotor sequence) and other nucleotide sequences. The regulatory sequence, by being "operatively linked", can regulate the transcription and/or decoding of the other nucleotide sequences.

The recombinant vector may be constructed typically as a cloning vector or an expression vector. For the expression vector, common vectors used for expressing exogenous proteins in plants, animals, or microorganisms may be used. The recombinant vector may be constructed through various methods known in the art.

The recombinant vector may be constructed with a prokaryotic cell or a eukaryotic cell as a host. For example, where the vector used is an expression vector and a prokaryotic cell is used as the host, it is common to include a robust promoter capable of advancing transcription (e.g., a CMV promoter, trp promoter, lac promoter, tac promoter, T7 promoter, etc.), a ribosome binding site for initiating translation, and a transcription/translation termination sequence. When a eukaryotic cell is used as the host, the origin of replication acting in the eukaryotic cell included in the vector includes f1 origin of replication, SV40 origin of replication, pMB1 origin of replication, adeno origin of replication, AAV origin of replication, and BBV origin of replication, but is not limited thereto. In addition, a promoter derived from a mammalian genome (e.g., a metallothionein promoter) or a promoter derived from a mammalian virus (e.g., an adenovirus late promoter, a vaccinia virus 7.5K promoter, an SV40 promoter, a cytomegalovirus promoter, and a tk promoter of HSV) may be used. In addition, a transcription termination sequence may in general include a polyadenylation sequence.

The above recombinant cell may be one obtained by introducing the above recombinant vector into an appropriate host cell. The host cell, which is capable of cloning or expressing the recombinant vector in a reliable and consecutive manner, may be any host cell known in the art. A prokaryotic host cell may be, for example, a Bacillus genus bacterium, such as E. coli JM109, E. coli BL21, E. coli RR1, E. coli LE392, E. coli B, E. coli X 1776, E. coli W3110, Bacillus subtilis, and Bacillus thuringiensis, or an intestinal bacterium, such as Salmonella typhimurium, Serratia marcescens, and various Pseudomonas species. A eukaryotic host cell may be, for example, a yeast (e.g., Saccharomyce cerevisiae), an insect cell, a plant cell, or an animal cell, for example, Sp2/0, Chinese hamster ovary (CHO) K1, CHO DG44, PER. C6, W138, BHK, COS-7, 293, HepG2, Huh7, 3T3, RIN, or an MDCK cell line.In addition, the MSCs and the PRL-1 or an active fragment thereof may be ones that reduce fat accumulation and/or fat tissue increase.

This may be achieved by reducing the expression of adipogenesis-associated factors. The present invention is defined by the claims. According to one aspect, provided is a pharmaceutical composition as defined in claims 4-6 for use in preventing or treating a disease associated with abnormal adipogenesis or abnormal fat metabolic dysfunction, the pharmaceutical composition comprising a mesenchymal stem cell which overexpresses PRL-1 compared to a non-genetically engineered parent cell.

Also described herein but not claimed is a health functional food for preventing or treating a disease associated with abnormal adipogenesis or abnormal fat metabolic dysfunction, the health functional food including a mesenchymal stem cell which overexpresses PRL-1 compared to a non-genetically engineered parent cell.

The term "disease associated with abnormal adipogenesis or abnormal fat metabolic dysfunction" as used in the present specification may refer to a disease caused by dysfunction in fat metabolism.

Examples of the disease associated with abnormal adipogenesis or abnormal fat metabolic dysfunction may include obesity, diabetes, dyslipidemia, metabolic diseases, hypertension, thyroid-associated ophthalmopathy, or degenerative diseases associated with abnormal adipogenesis or abnormal fat metabolic dysfunction. The term "thyroid-associated ophthalmopathy (TAO)" as used in the present specification refers to inflammatory orbitopathy caused by hyperthyroidism caused by over-secretion of thyroid hormones.

The TAO may manifest disease symptoms such as eyelid retraction, proptosis, restrictive strabismus, reduced eyesight, double vision, and the like. The term "treatment" as used herein refers to or includes alleviating, inhibiting the progress of, or preventing a disease, a disorder, or a disease condition, or one or more symptoms thereof. It is understood that the present invention does not provide a method of treatment but a pharmaceutical composition for use in such treatment. The terms "active ingredient" or "pharmaceutically effective amount" as used herein may refer to any amount of a composition for use according to the present disclosure that is sufficient for alleviating, inhibiting the progress of, or preventing a disease, a disorder, or a disease condition, or one or more symptoms thereof.

As used herein, the terms "administered," "introduced", and "implanted" are used interchangeably and may refer to the placement of a composition according to a specific example into a subject by a method or route which results in at least a partial localization of the composition according to the specific example at a desired site. The administration is not encompassed by the scope of the invention.

At least a portion of cells or cellular components of a composition according to a specific example may be administered by any suitable route capable of delivering the same to a desired site in a living subject. Once administered to a subject, the cells may have a survival time of from several hours at the shortest, for example, 24 hours, up to several days or several years at the longest. The composition may include PRL-1 or an active fragment thereof, in an amount of 0.001 wt% to 80 wt% with respect to the total weight of the composition.

In addition, the administration dose of the PRL-1 or an active fragment thereof may be 0.01 mg to 10,000 mg, 0.1 mg to 1,000 mg, 1 mg to 100 mg, 0.01 mg to 1,000 mg, 0.01 mg to 100 mg, 0.01 mg to 10 mg, or 0.01 mg to 1 mg. In addition, the administration dose of MSCs may be 1.0 x 10⁵ cells/kg to 1.0 x 10⁸ cells/kg (body weight). However, the administration dose may be prescribed in various amounts depending on a number of factors, i.e., a preparation method, a manner of administration, a patient's age, body weight, sex, severity of the disease, diet, administration time, administration route, rate of excretion, and reactive sensitivity, and those skilled in the art may appropriately adjust the administration dose by taking such factors into consideration. In terms of administration frequency, the administration may be made once, or within a range of clinically acceptable side effects, may be made twice or more, and also in terms of administration site, the administration may be made at a single site or at two or more sites. Further, for non-human animals as well, the administration may be made at the same administration dose per kg as a human, or alternatively for example, may be made at a dose that is converted from the above administration dose by using the volume ratio (for example, a mean value) of an organ (e.g., the heart) between a target animal and the human. Possible administration routes may include oral, subglossal, non-oral (for example, subcutaneous, intramuscular, intra-arterial, intraperitoneal, intradural or intravenous), rectal, local (including percutaneous) routes, inhalation, and injection, or may include insertion of a material or an implantable device. An animal subjected to treatment according to a specific example may be a human and any other desired mammal, and more specifically, includes a human, a monkey, a mouse, a rat, a rabbit, a sheep, a cow, a dog, a horse, a pig, and the like. A pharmaceutical composition according to a specific example may comprise a pharmaceutically acceptable carrier and/or an additive.

For example, the pharmaceutical composition may comprise sterile water, a physiological saline, a known buffer (phosphoric acid, citric acid, and other organic acids, etc.), a stabilizer, a salt, an antioxidant (e.g., ascorbic acid), a surfactant, a suspension, an isotonic agent, or a preservative. For local administrations, the pharmaceutical composition may be combined with an organic material such as a biopolymer, or an inorganic material such as hydroxyapatite, and more specifically, may be combined with a collagen matrix, a polylactic acid complex or copolymer, a polyethyleneglycol polymer or copolymer, or chemical derivatives thereof, or the like. The pharmaceutical composition according to a specific example, when prepared as a formulation suitable for injection, may have the MSCs or PRL-1 dissolved in a pharmaceutically acceptable carrier, or may be frozen as a solution state with the MSCs or PRL-1 dissolved therein. The pharmaceutical composition according to a specific embodiment, if needed for its mode of administration or formulation, may appropriately include a suspension, a solubilizing agent, a stabilizing agent, an isotonic agent, a preservative, an anti-adhesion agent, a surfactant, a diluent, an excipient, a pH adjustment agent, a pain-reducing agent, a buffer, a reducing agent, an antioxidant, or the like.

In addition to the examples disclosed above, pharmaceutically acceptable carriers and agents suitable for the present disclosure are disclosed in detail in literature [Remington's Pharmaceutical Sciences, 19th ed., 1995]. The pharmaceutical composition according to a specific example may be formulated using a pharmaceutically acceptable carrier and/or an excipient and prepared as a unit dosage form, or may be injected and prepared in a multi-dose container, according to a method that can be easily enabled by a person skilled in the art in the technical field to which the present disclosure belongs. In detail, the formulation may be a solution in an oil or water medium, a suspension, or an emulsion, or may be in a form of powder, granules, pills, or capsules. The health functional food may be used in combination with other food or food ingredients, in addition to the PRL-1 or an active fragment thereof, and may be used appropriately according to a known method. A mixing amount of an active ingredient may be appropriately determined in accordance with an intended purpose of use (prevention, health, or therapeutic treatment).

In general, when preparing a health functional food, the composition of the present specification may be added in an amount of 15 parts by weight or less with respect to raw materials. The type of the health functional food is not particularly limited. According to another aspect, provided is a method of inhibiting fat accumulation, the method including: administering MSCs to a test animal in vivo or contacting the MSCs with cells in vitro, the MSCs being engineered, by a culture environment or genetically, to overexpress PRL-1 compared to a non-genetically engineered parent cell. In the method of inhibiting fat accumulation, the contact may refer to treating or contacting a subject with the MSCs, PRL-1 or an active fragment thereof.

The treatment or contact may include co-culturing the MSCs with the cells, or administering the MSCs, PRL-1 or an active fragment thereof, to a test animal, for example, to a specific organ or periorbital tissues of the animal by direct local injection or intravenous injection (i.v. injection).

Further, the treatment or contact may include contacting, for example, co-culturing host cells including a nucleotide sequence encoding PRL-1 or an active fragment thereof with the cells, or administering the host cells to a test animal, for example, to a specific organ or periorbital tissues of the animal by direct location injection, i.v. injection, or the like. Local injection to periorbital tissues may be effective as an ideal administration method for treatment of ophthalmologic disease. The co-culturing and administration may be performed by a method and over a period of time that are sufficient for those skilled in the art to achieve a desired effect, for example, until the MSCs, or PRL-1 or an active fragment thereof can exert influence on the subject. ADVANTAGEOUS EFFECTS OF DISCLOSURE Mesenchymal stem cells, or a composition including the same can be advantageously utilized in the prevention, treatment, or alleviation of a disease associated with abnormal fat metabolism by inhibiting fat accumulation or adipose tissue increase.

### BRIEF DESCRIPTION OF DRAWINGS

FIG. 1 is a schematic diagram illustrating a PRL-1 gene transferred into mesenchymal stem cells.
FIG. 2 is a microscopic photograph confirming, through GFP, whether the PRL-1 gene has been transferred into placenta-derived mesenchymal stem cells.
FIG. 3 is a graph showing results of confirming the number of PRL-1 gene-transferred placenta-derived mesenchymal stem cells (GFP+ cells).
FIG. 4 illustrates results of confirming, through RT-PCR, an mRNA expression level of PRL-1 expressed by mesenchymal stem cells according to one aspect.
FIG. 5 illustrates results of confirming, through RT-PCR, expression levels of cell surface antigens (Oct4, Nanog, Sox2, HLA-G, TERT) of mesenchymal stem cells according to one aspect.
FIG. 6 illustrates results of confirming, through ELISA, expression levels of cell surface antigens (Oct4, HLA-G) of mesenchymal stem cells according to one aspect.
FIG. 7 is a graph confirming a doubling time of mesenchymal stem cells according to one aspect.
FIGS. 8 and 9 illustrate results of analyzing, through FACS, the characteristics of cell surface antigens (CD34, CD13, CD90, CD105, HLA-DR, HLA-ABC, and HLA-G) of mesenchymal stem cells according to one aspect.
FIG. 10 illustrates results of confirming the expression of cell surface antigen markers and osteocyte and adipocyte markers in placenta-derived MSCs with enhanced PRL-1 expression when induced to differentiate to osteocytes and adipocytes.
FIG. 11 illustrates results of confirming, through co-culture, the effect of mesenchymal stem cells according to one aspect on adipogenesis of orbital fibroblasts; OF: Normal subject; TAO: Subject with thyroid-associated ophthalmopathy; CP(-): Not co-cultured; CP Naive (+): Co-cultured with placenta-derived mesenchymal stem cells; and CP-PRL-1 (+): Co-cultured with placenta-derived mesenchymal stem cells overexpressing CP-PRL-1.
FIG. 12 illustrates results of confirming, through co-culture, the ability of mesenchymal stem cells according to one aspect to regulate adipogenesis-associated genes of orbital fibroblasts; OF: Normal subject; and TAO: Subject with thyroid-associated ophthalmopathy.
FIG. 13 illustrates results of confirming, through co-culture, the influence of mesenchymal stem cells according to one aspect on adipogenesis-associated factors and inflammation-associated factors of orbital fibroblasts; TAO: Subject with thyroid-associated ophthalmopathy.

### MODE OF DISCLOSURE

Hereinafter, the present disclosure will be described in greater detail by way of examples.

However, the following examples are only for illustrating one or more specific examples, and the scope of the present disclosure is not limited to the following examples.

### Reference Examples

### Reference example 1. Culturing and Treatment of Orbital Fibroblasts

Orbital fibroblasts were collected from human subjects (4 normal subjects, 4 patients) and cultured in DMEMF12 (Gibco) (10% FBS and 1% penicillin-streptomycin). 2 days after the fibroblasts were distributed and grown in media, 5 µg/ml of insulin, 1 mM dexamethasone, and 0.5 mM IBMX were added to DMEM (10% FBS) to initiate differentiation to adipocytes (day 0).

After 72 hours (day 3), the media were switched with DMEM media supplemented with 10% FBS and 5 µg/ml insulin, and DMEM media supplemented with 10% FBS were fed every two days thereafter.

Reference example 2. Real-Time PCR On day 8 of culturing, placenta-derived mesenchymal stem cells with overexpressed PRL-1 (2 x 10⁵) were co-cultured with orbital fibroblasts for 48 hours.

Cell lysates were homogenized using the Trizol Reagent (Invitrogen, Carlsbad, CA, USA) to extract RNA. From each sample, 1 µg of total RNA was reverse-transcribed to synthesize cDNA. The cDNA synthesis conditions were as follows: RNA denaturation (65 °C, 1 minute), unwinding (25 °C, 5 minutes), amplification (42 °C, 60 minutes), and enzyme deactivation (85 °C, 1 minute).

The mRNA expression of each gene was amplified under the following PCR conditions and normalized: initial denaturation (95 °C, 2 minutes), amplification (95 °C, 10 seconds; 55 °C, 20 seconds; and 72 °C, 20 seconds), 40 cycles. PPARγ, ADIPONECTIN, C/EBPα, primer sets used are shown in Table 1.

**[Table 1] Markers Genes Primer sequence (5'-3')**

| Ligation temperature (°C) SEQ ID NO. | | | | |
|---|---|---|---|---|
| Stemness | | | | |
| 04-Oct | F: AGT GAG AGG CAA CCT GGA GA | 52 | 1 | R: GTG AAG TGA GGG CTC CCA TA |
| 2 | Nanog | F: TTC TTG ACT GGG ACC TTG TC | 52 | 3 |
| | | R: GCT TGC CTT GCT TTG AAG CA | | 4 |
| | Sox-2 | F: AGA ACC CCA AGA TGC ACA AC | 52 | 5 |
| | | R: GGG CAG CGT GTA CTT ATC CT | | 6 |
| | HLA-G | F: GCG GCT ACT ACA ACC AGA GC | 58 | 7 |
| | | R: GCA CAT GGC ACG TGT ATC TC | | 8 |
| | TERT | F: GAG CTG ACG TGG AAG ATG AG | 55 | 9 |
| | | R: CTT CAA GTG CTG TCT GAT TCC AAT G | | 10 |
| | Osteocyti c | OC | F: CAC TCC TCG CCC TAT TGG C | 58 |
| | | 11 | | R: CCC TCC TGC TTG GAC ACA AAG |
| 12 | Col 1 | F: AGA CAT CCC ACC AAT CAC CT | 60 | 13 |
| | | R: CGT CAT CGC ACA ACA CCT | | 14 |
| | Adipocytic | Adipsin | F: CAC GTA CCA TGA TGG GGC AA | 55 |
| | | 15 | | R: TCG AGA TCC CCA CGT AAC CA |
| 16 | PPARg | F: GAC AGA CCT CAG GCA GAT TG | 55 | 17 |
| | | R: GTC AGC GAC TGG GAC TTT TC | | 18 |
| | Adiponect in | F: GAC TGC CAC TAA TTC AGA GC | 55 | 19 |
| | | R: CTC ATG GGG ATA ACA CTC AG | | 20 |
| | LPL | F: ACA GGT GCA ATT CCA AGG AG | 55 | 21 |
| | | R: CTT TCA GCC ACT GTG CCA TA | | 22 |
| | Leptin | F: ATC TAT GTG CAC CTG AGG GTA G | 55 | 23 |
| | | R: TCC TTT TCA CAA AGC CAC ACT AT | | 24 |
| | FABP4 | F: ACA TGA AAG AAG TGG GAG TTG GC | 55 | 25 |
| | | R: AAG TAC TCT CTG ACC GGA TGA CG | | 26 |
| | C/EBPα | F: TGT ATA CCC CTG GTG GGA GA | 55 | 27 |
| | | R: TCA TAA CTC CGG TCC CTC TG | | 28 |
| | - | PRL-1 | F: TAC TGC TCC ACC AAG AAG CC | 60 |
| | | 29 | | R: AGG TTT ACC CCA TCC AGG TC |
| 30 | Internal control group | human GAPDH | F: GCA CCG TCA AGG CTG AGA AC | 55 |
| | | 31 | | R: GTG GTG AAG ACG CCA GTG GA |
| 32 | rat GAPDH | F: TCC CTC AAG ATT GTC AGC AA | 55 | 33 |
| | | R: AGA TCC ACA ACG GAT ACA TT | | 34 |
| | The mRNA expressio ns of the respective genes were normalize d to 18s rRNA. | Data was compared to a normal group and expressed as folds (mean ± SEM) of an adipose differentiation-associated factor. | Reference Example 3. Western Blot | Using RIPA buffer, lysates were prepar ed. |
| | | Equivalent amounts of total proteins were separated by SDS-PAGE and transferred to a membrane. | | The membr ane was diluted to 1:1,000 in anti-HAS1 and HAS2 (Santa Cruz Biotech nology, SA, USA) and subject ed to immun oblottin g, and the same membr ane was culture d with GAPD H (Santa Cruz). |

After rinsing, the membrane was diluted to 1:1,000 and cultured at room temperature for 3 hours with horseradish peroxidase-conjugated anti-goat IgG secondary antibody. Immunoreactive bands were imaged with an enhanced chemiluminescence solution (Animal Genetics, Suwon, Korea) and detected with ChemiDocTM XRS+ System Imager (Bio-Rad Laboratories, Hercules, CA, USA).

Protein expression amounts were normalized to GAPDH. Data was compared to a normal group and expressed as folds of HAS2 (mean ± SEM).

Reference example 4. FACS Analysis Human fibroblasts (3 x 10⁵) were dissociated in a cell dissociation buffer (Life Technologies) and rinsed with PBS (2%(v/v) FBS). The resulting cells were cultured with an isotype control IgG or an antigen-specific antibody (BD Biosciences, CA, USA) for 20 minutes, and used to identify cells. FACS sorting was performed using the FACS vantage Flow Cytometer (BD Biosciences, CA, USA). Example 1. Preparation of Functionally-Enhanced Mesenchymal Stem Cells 1.1. Isolation of Placenta-Derived Mesenchymal Stem Cells After obtaining an informed consent from a healthy mother who had given a normal birth, tissues were isolated from placental tissues collected from the placenta at the time of the normal birth.

The isolated tissues (chorioamniotic membranes) were placed in a 50 ml tube and had the remnant blood removed therefrom with the addition of DPBS, and subsequently, suspended matter was collected to one side by scratching the inner lining of the chorioamniotic membranes with a slide glass sterilized in 20 ml of enzyme solution I (1 mg/ml collagenase type I, 2 mg/ml Trypsin, 20 mg/ml DNase I, 1.2 U/ml Dispase, x1 PS in HBSS). After adding 10 ml of enzyme solution I and thoroughly mixing the resulting solution, enzymatic reactions were twice repeated each for 15 minutes to separate stem cells from the tissues.

The isolated cell suspension was separated by centrifugation, and the isolated cells were cultured using DMEM/F12 supplemented with 10% fetal bovine serum, 1 ug/ml heparin, and 25 ng/ml fibroblast growth factor-4 (FGF-4). Subsequently, the culture media were changed every 4 to 5 days, and subculturing was performed by treating the first subculture with TrypLE, manufactured by Invitrogen, in a short time (3 minutes) in an incubator at 37 °C. 1.2. Preparation of Placenta-Derived Mesenchymal Stem Cells with Enhanced Expression of PRL-1 Gene Electroporation was used to enhance the expression of PRL-1 gene in the placenta-derived mesenchymal stem cells isolated in section 1.1. above.

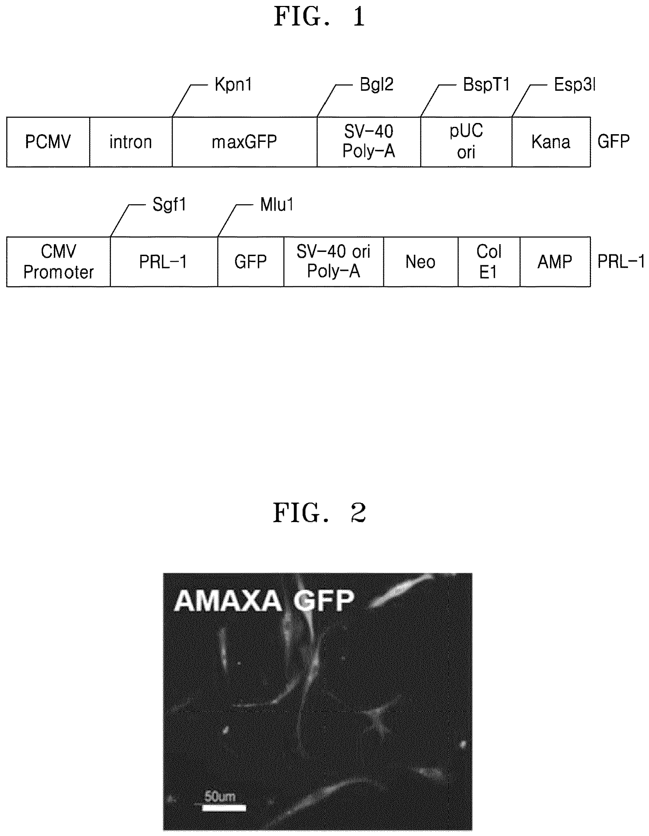

In detail, a gene having the structure shown in FIG. 1 was transferred into the mesenchymal stem cells by means of electroporation. Chorionic plate-derived mesenchymal stem cells (CP-MSCs) were cultured in MEM-alpha media supplemented with 10% fetal bovine serum (FBS), 1% penicillin-streptomycin, 25 ng/ml FGF-4, and 1 ug/ml heparin, in an incubator at 37 °C under CO₂.

Cells were rinsed with phosphatase buffered saline (PBS) and then treated with trypsin at 37 °C for 2 minutes to detach the cells, and the cells detached using the PBS were collected and subjected to centrifugation at 1,200 rpm for 5 minutes. The pellets thus obtained were suspended with the addition of 1 ml of culture broth, and cell counting was performed using a hemocytometer.

After subjecting the collected cells to centrifugation at 200 g for 10 minutes and adding Nucleofector to suspend the cells (5 x 10⁵/100ul), 2 ug of DNA plasmids containing PRL-1 was added. The cells were transferred into a cuvette and placed into a Nucleofection machine, and a 'U-23' program was run. Immediately upon termination of the program, a culture dish containing new media was stabilized in the 37 °C, CO₂ incubator. After 4 hours, the media in the culture dish were removed using an aspirator, and the attached cells were cultured in media containing 1.5 mg/ml neomycin (10% FBS) and in MEM-alpha media supplemented with 1% penicillin-streptomycin, 25 ng/ml FGF-4, and 1 ug/ml heparin, to yield PRL-1 gene-transferred CP-MSCs.

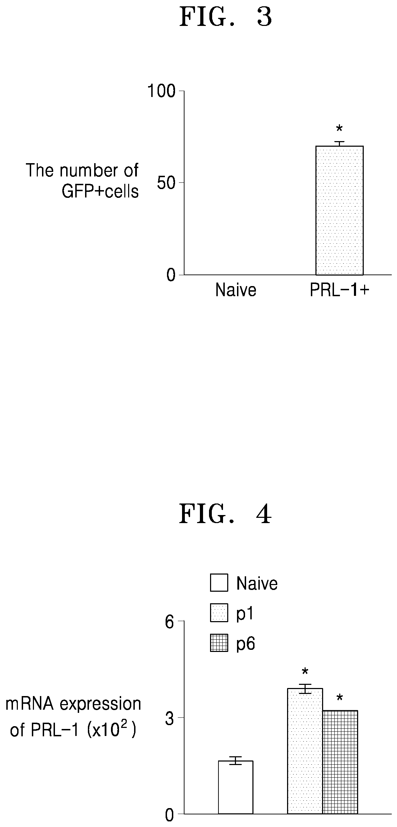

FIG. 2 is a microscopic photograph confirming, using GFP, whether the PRL-1 gene had been transferred into placenta-derived mesenchymal stem cells. FIG. 3 is a graph showing a result of confirming the number of PRL-1 gene-transferred placenta-derived mesenchymal stem cells (GFP+ cells).

As shown in FIG. 2 and FIG. 3, it was found that there was a significant increase in expression of the PRL-1 gene artificially transferred into the enhanced placenta-derived mesenchymal stem cells prepared in section 1.1., compared to in naive placenta-derived mesenchymal stem cells serving as a control group with no transferred PRL-1 gene.

Therefore, it was confirmed that the placenta-derived mesenchymal stem cells with enhanced expression of PRL-1 gene were successfully produced by the method described in section 1.1.

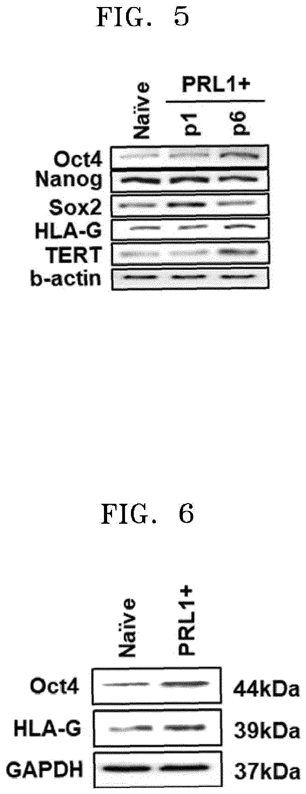

1.3. Analysis of Characteristics of Placenta-Derived Mesenchymal Stem Cells with Enhanced Expression of PRL-1 Gene To analyze the characteristics of mesenchymal stem cells confirmed in sections 1.1. and 1.2. above, the characteristics of cytokine secretions and the characteristics of cell surface antigens were analyzed. In detail, the expression level of PRL-1 in the placental-derived mesenchymal stem cells was confirmed through RT-PCR analysis, and results thereof are shown in FIG. 4. FIG. 4 shows the mRNA expression level of PRL-1 expressed in cells. As shown in FIG. 4, the results of RT-PCR analysis showed that the placenta-derived mesenchymal stem cells (p1, p6) prepared in section 1.1. had a significantly higher mRNA expression level of PRL-1, compared to the naive cells. In addition, the expression levels of cell surface antigens (Oct4, Nanog, Sox2, HLA-G, and TERT) in the placenta-derived mesenchymal stem cells were confirmed through RT-PCR analysis, and results thereof are shown in FIG. 5. In addition, the expression levels of cell surface antigens (Oct4, HLA-G) in the placenta-derived mesenchymal stem cells were confirmed through ELISA analysis, and results thereof are shown in FIG. 6.

As shown in FIGS. 5 and 6, RT-PCR and ELISA analyses demonstrated that the placenta-derived mesenchymal stem cells (PRL1+) prepared in section 1.1. expressed the stem cell markers, Oct4, Nanog, Sox2, HLA-G, and TERT.

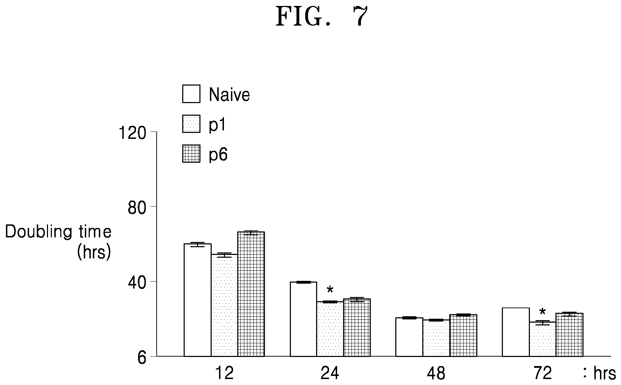

In addition, a doubling time at which the number of cells were doubled was confirmed by counting increases in the number of cells over cell culture processes, and results thereof are shown in FIG. 7.

As shown in FIG. 7, it was found that there was no large difference in terms of the doubling time over sub-culture processes between the PRL-1 overexpressed cells and the control group that was not induced for PRL-1 expression.

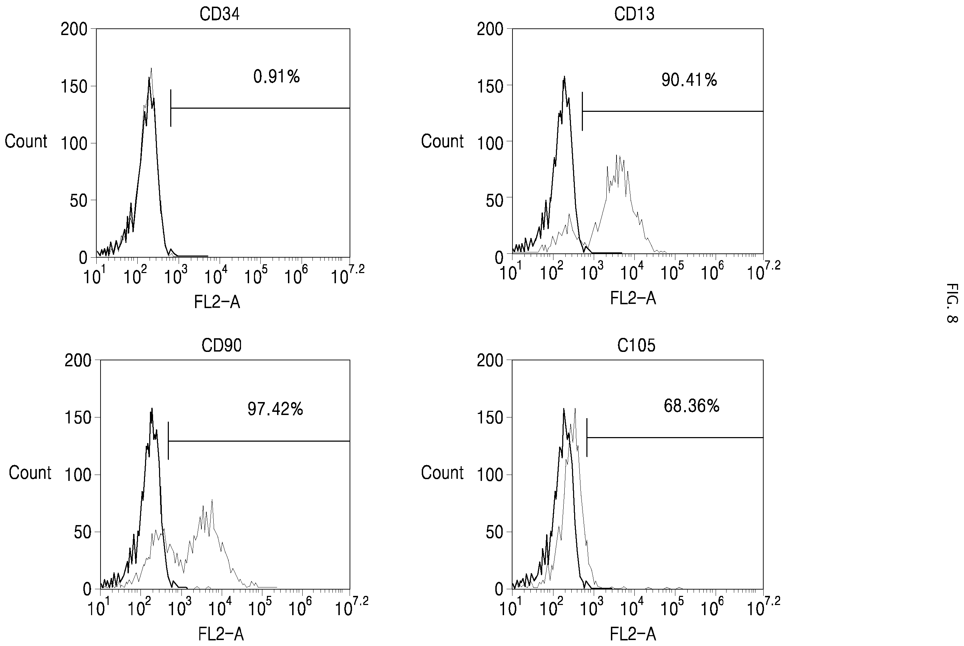

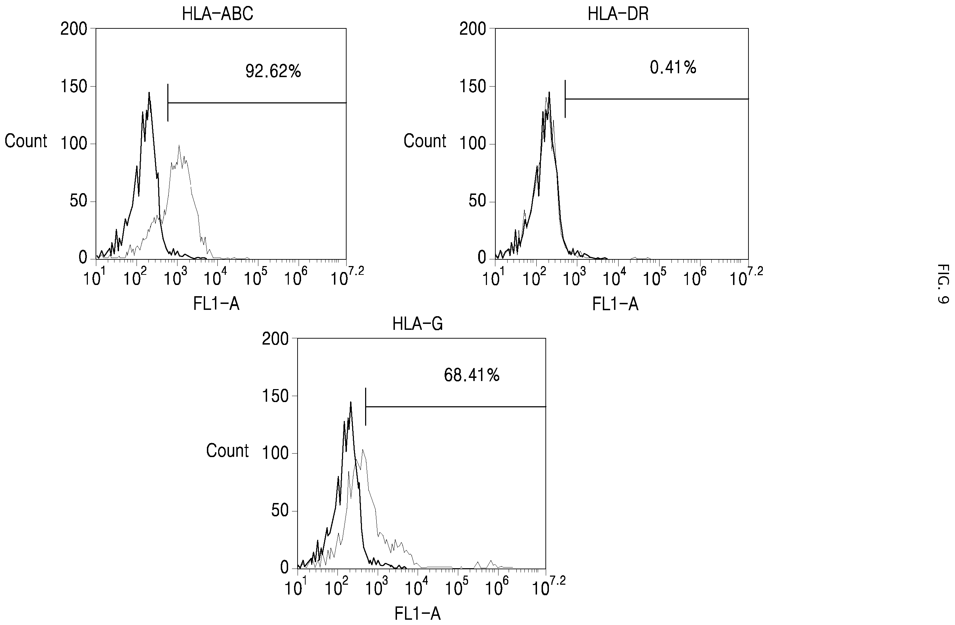

Further, the characteristics of cell surface antigens (CD34, CD13, CD90, CD105, HLA-DR, HLA-ABC, and HLA-G) of the placenta-derived mesenchymal stem cells were analyzed through FACS analyses, and results thereof are shown in FIGS. 8 and 9.

As shown in FIGS. 8 and 9, the mesenchymal stem cell markers, CD34, CD105, HLA-ABC, and HLA-G were found to be positive. Accordingly, it was confirmed that as the expression of PRL-1 was enhanced, the placenta-derived mesenchymal stem cells, which express and include PRL-1, had maintained the characteristics of stem cells.

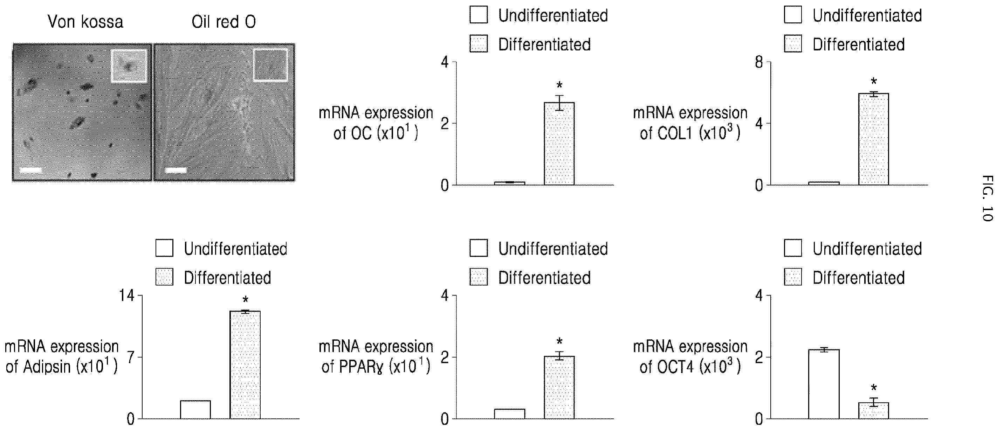

In addition, FIG. 10 shows results of confirming the expression of cell surface antigen markers and osteocyte and adipocyte markers in the placenta-derived mesenchymal stem cells with enhanced expression of PRL-1, when induced to differentiate to osteocytes and adipocytes. As shown in FIG. 10, it was confirmed that when induced to differentiate to osteocytes or adipocytes, as the expression of the undifferentiation marker Oct4 decreases, the expressions of genes, such as osteocyte and adipocyte markers, i.e., osteocalsin (OC), type I collagen (Col 1), adipsin, and PPAR-r, were increased, thus demonstrating a successful differentiation to osteocytes and adipocytes. Example 3. Confirmation of Adipogenesis Inhibition

Assessment was made on whether the above-prepared placenta-derived mesenchymal stem cells with enhanced expression of PRL-1 had inhibitory effects on adipogenesis of orbital fibroblasts. In detail, orbital fibroblasts isolated from normal subjects and subjects with thyroid-associated ophthalmopathy (TAO) were placed in adipogenic induction differentiation media and cultured for 10 days, with or without the placenta-derived MSCs with enhanced expression of PRL-1 prepared above (CP-PRL-1) co-cultured therewith (for first 4 days in DMEM supplemented with 10% FBS, 33 uM biotin, 17 uM pantothenic acid, 0.2 nM T3, 10 µg/mL transferrin, 0.2 uM prostaglandin I2, 0.1 mM isobutylmethylxanthine (IBMX), 1 uM dexamethasone, and 5 ug/ml insulin / from 5-10 days w/o IBMX, dexamethaxone, insulin).

After 10 days, changes in fat accumulation in the fibroblasts were observed through Oil-Red-O staining.

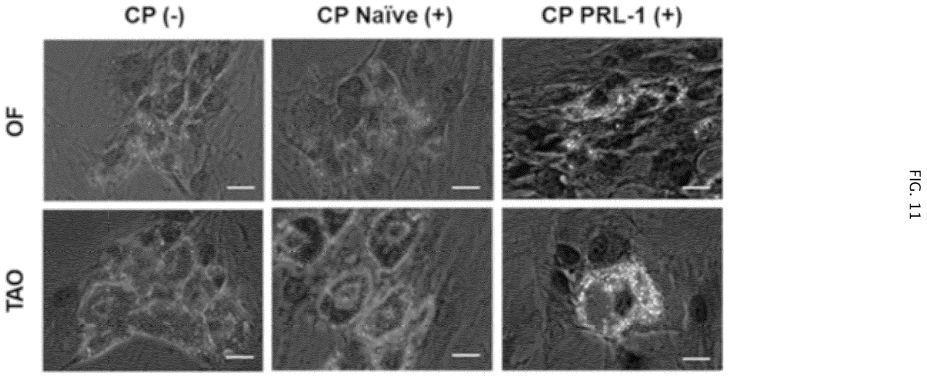

The result of observation is shown in FIG. 11.

OF: Normal; TAO: Subject with thyroid associated ophthalmopathy; CP(-): Not co-cultured; CP Naive (+): Co-cultured with placenta-derived mesenchymal stem cells; CP-PRL-1 (+): Co-cultured with CP-PRL-1.

As a result, as shown in FIG. 11, it was found that fat accumulation in fibroblasts of the TAO patients, induced in differentiation media, was significantly decreased through co-culture with CP-PRL-1. Example 4. Confirmation of Ability to Regulate Adipogenesis-Associated Genes

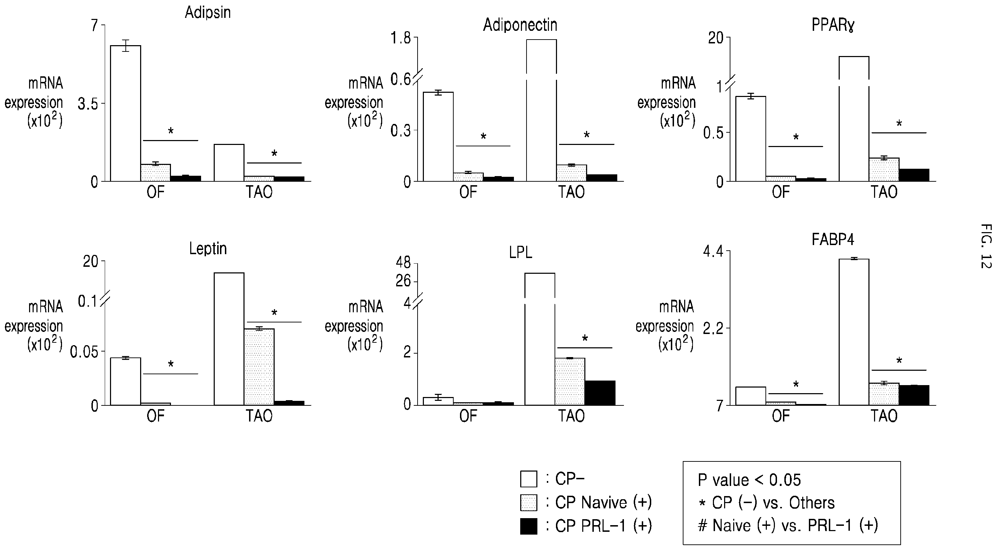

To assess whether the placenta-derived mesenchymal stem cells with enhanced expression of PRL-1 prepared above have the ability to regulate adipogenesis-associated genes of orbital fibroblasts, orbital fibroblasts were not co-cultured with CP-PRL-1 (CP-), co-cultured with naive mesenchymal stem cells (CP Naive (+)), or co-cultured with CP-PRL-1 (CP-PRL-1 (+)), and mRNA levels of the adipogenesis-associated genes (adipsin, adiponectin, PPARγ, leptin, LPL, FABP4) expressed in these orbital fibroblasts were measured through qRT-PCR. Results thereof are shown in FIG. 12.

OF: Normal subject; TAO: Subject with thyroid associated ophthalmophathy.

As shown in FIG. 12, there was a decrease in expression level of all the adipogenesis-associated genes in the orbital fibroblasts co-cultured with the mesenchymal stem cells.

In particular, it was found that the expression levels of the adipogenesis-associated genes were significantly decreased when co-cultured with CP-PRL-1, compared to when co-cultured with naive mesenchymal stem cells.

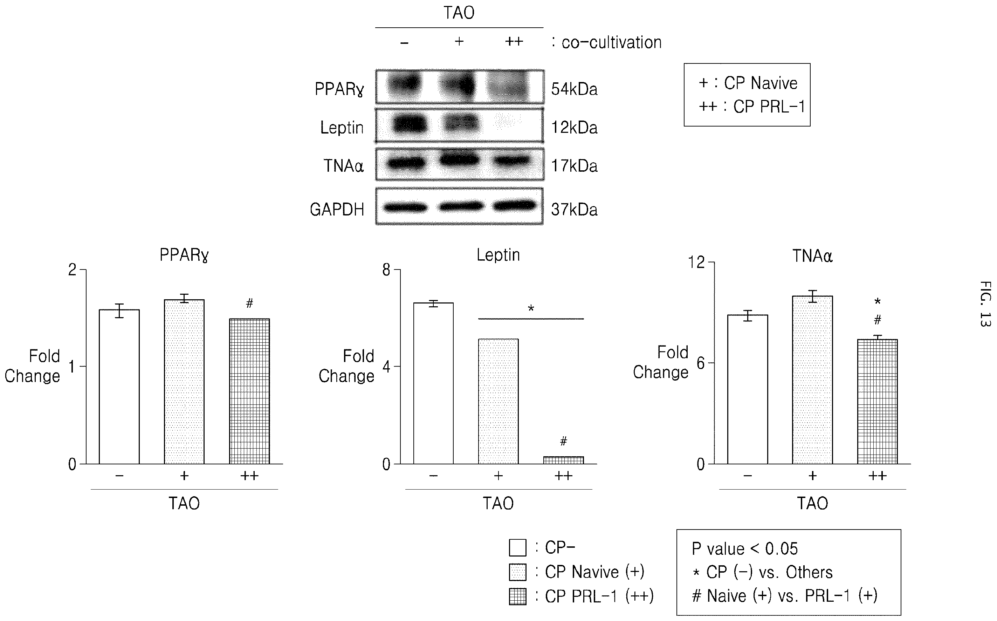

In addition, in the above experiment groups, the gene expression levels of adipogenesis-associated factors (leptin, PPARγ) and the inflammation-associated factor TNF-α were confirmed through qRT-PCR, and their protein expression levels were confirmed by western blot. Results thereof are shown in FIG. 13.

TAO: Subject with thyroid associated ophthalmopathy. As shown in FIG. 13, it was found that when co-cultured with CP-PRL-1, there was a significant decrease in expression level of genes and proteins of adipogenesis-associated factors and inflammation-associated factors, compared to when co-cultured with naive mesenchymal stem cells. Accordingly, the placenta-derived mesenchymal stem cells with enhanced expression of PRL-1 have a desirable effect of inhibiting adipogenesis, and thus may be advantageously used as a therapeutic agent for diseases associated with adipogenesis and/or fat accumulation.

## Claims

1. A mesenchymal stem cell overexpressing PRL-1 compared to a non-genetically engineered parent cell.

2. The mesenchymal stem cell of claim 1, wherein the PRL-1 is transferred by a method and expressed, the method being selected from microinjection, electroporation, a DEAE-dextran treatment transfection method, lipofection, a nanoparticle-mediated transfection method, a protein transduction domain-mediated transfection method, a calcium phosphate (CaPO4) transfection method, a virus-mediated gene transfer method, and a PEG-mediated transfection method.

3. The mesenchymal stem cell of claim 1, wherein the mesenchymal stem cell is an umbilical cord-derived mesenchymal stem cell, an umbilical cord blood-derived mesenchymal stem cell, a bone marrow-derived mesenchymal stem cell, a placenta-derived mesenchymal stem cell, or an adipose-derived mesenchymal stem cell.

4. A pharmaceutical composition comprising a mesenchymal stem cell overexpressing PRL-1 compared to a non-genetically engineered parent cell, for use in treating or preventing a disease associated with abnormal adipogenesis or abnormal fat metabolic dysfunction by inhibiting adipogenesis, wherein the disease associated with abnormal adipogenesis or abnormal fat metabolic dysfunction is selected from obesity, diabetes, dyslipidemia, metabolic diseases, hypertension, thyroid-associated ophthalmopathy, and degenerative diseases associated with abnormal adipogenesis or abnormal fat metabolic dysfunction.

5. The pharmaceutical composition for use of claim 4, wherein the mesenchymal stem cell inhibits fat accumulation or adipose tissue increase.

6. The pharmaceutical composition for use of claim 4, wherein the mesenchymal stem cell is an umbilical cord-derived mesenchymal stem cell, an umbilical cord blood-derived mesenchymal stem cell, a bone marrow-derived mesenchymal stem cell, a placenta-derived mesenchymal stem cell, or an adipose-derived mesenchymal stem cell.

7. A method of inhibiting fat accumulation, the method comprising: contacting a mesenchymal stem cell with cells *in vitro,* the mesenchymal stem cell overexpressing PRL-1 compared to a non-genetically engineered parent cell.

## Patentansprüche

1. Mesenchymale Stammzelle, die PRL-1 im Vergleich zu einer nicht gentechnisch veränderten Mutterzelle überexprimiert.

2. Mesenchymale Stammzelle nach Anspruch 1, wobei das PRL-1 durch ein Verfahren übertragen und exprimiert wird, wobei das Verfahren ausgewählt ist aus Mikroinjektion, Elektroporation, einem DEAE-Dextran-Behandlungs-Transfektionsverfahren, Lipofektion, einem Nanopartikel-vermittelten Transfektionsverfahren, einem Protein-Transduktionsdomänen-vermittelten Transfektionsverfahren, einem Calciumphosphat (CaPO4)-Transfektionsverfahren, einem Virus-vermittelten Gentransferverfahren und einem PEG-vermittelten Transfektionsverfahren.

3. Mesenchymale Stammzelle nach Anspruch 1, wobei die mesenchymale Stammzelle eine aus Nabelschnur stammende mesenchymale Stammzelle, eine aus Nabelschnurblut stammende mesenchymale Stammzelle, eine aus Knochenmark stammende mesenchymale Stammzelle, eine aus Plazenta stammende mesenchymale Stammzelle oder eine aus Adiposa stammende mesenchymale Stammzelle ist.

4. Pharmazeutische Zusammensetzung, die eine mesenchymale Stammzelle umfasst, die PRL-1 im Vergleich zu einer nicht gentechnisch veränderten Mutterzelle überexprimiert, zur Verwendung bei der Behandlung oder Vorbeugung einer Erkrankung, die mit einer abnormalen Adipogenese oder einer abnormalen Fettstoffwechsel-Dysfunktion verbunden ist, durch Hemmung der Adipogenese, wobei die mit abnormaler Adipogenese oder abnormaler Fettstoffwechsel-Dysfunktion verbundene Erkrankung ausgewählt ist aus Fettleibigkeit, Diabetes, Dyslipidämie, Stoffwechselkrankheiten, Bluthochdruck, Schilddrüsen-assoziierter Ophthalmopathie und degenerativen Krankheiten, die mit abnormaler Adipogenese oder abnormaler Fettstoffwechsel-Dysfunktion verbunden sind.

5. Pharmazeutische Zusammensetzung zur Verwendung nach Anspruch 4, wobei die mesenchymale Stammzelle die Fettansammlung oder die Zunahme des Fettgewebes hemmt.

6. Pharmazeutische Zusammensetzung zur Verwendung nach Anspruch 4, wobei die mesenchymale Stammzelle eine aus Nabelschnur stammende mesenchymale Stammzelle, eine aus Nabelschnurblut stammende mesenchymale Stammzelle, eine aus Knochenmark stammende mesenchymale Stammzelle, eine aus Plazenta stammende mesenchymale Stammzelle oder eine aus Adiposa stammende mesenchymale Stammzelle ist.

7. Verfahren zur Hemmung der Fettansammlung, wobei das Verfahren umfasst: Inkontaktbringen einer mesenchymalen Stammzelle mit Zellen *in vitro,* wobei die mesenchymale Stammzelle PRL-1 im Vergleich zu einer nicht gentechnisch veränderten Mutterzelle überexprimiert.

## Revendications

1. Cellule souche mésenchymateuse surexprimant PRL-1 comparée à une cellule mère non modifiée génétiquement.

2. Cellule souche mésenchymateuse selon la revendication 1, où la PRL-1 est transférée par un procédé et exprimée, le procédé étant choisi parmi la microinjection, l'électroporation, un procédé de transfection par traitement au DEAE-dextrane, la lipofection, un procédé de transfection à médiation par des nanoparticules, un procédé de transfection à médiation par un domaine de transduction de protéine, un procédé de transfection au phosphate de calcium (CaPO4), un procédé de transfert de gène à médiation par un virus et un procédé de transfection à médiation par le PEG.

3. Cellule souche mésenchymateuse selon la revendication 1, où la cellule souche mésenchymateuse est une cellule souche mésenchymateuse dérivée du cordon ombilical, une cellule souche mésenchymateuse dérivée du sang du cordon ombilical, une cellule souche mésenchymateuse dérivée de la moelle osseuse, une cellule souche mésenchymateuse dérivée du placenta ou une cellule souche mésenchymateuse dérivée du tissu adipeux.

4. Composition pharmaceutique comprenant une cellule souche mésenchymateuse surexprimant PRL-1 comparée à une cellule mère non modifiée génétiquement, destinée à être utilisée dans le traitement ou la prévention d'une maladie associée à une adipogenèse anormale ou à un dysfonctionnement métabolique des graisses anormal par inhibition de l'adipogenèse,
où la maladie associée à une adipogenèse anormale ou à un dysfonctionnement métabolique des graisses anormal est choisie parmi l'obésité, le diabète, la dyslipidémie, les maladies métaboliques, l'hypertension, l'ophtalmopathie associée à la thyroïde et les maladies dégénératives associées à une adipogenèse anormale ou à un dysfonctionnement métabolique des graisses anormal.

5. Composition pharmaceutique destinée à être utilisée selon la revendication 4, où la cellule souche mésenchymateuse inhibe l'accumulation des graisses ou l'augmentation du tissu adipeux.

6. Composition pharmaceutique destinée à être utilisée selon la revendication 4, où la cellule souche mésenchymateuse est une cellule souche mésenchymateuse dérivée du cordon ombilical, une cellule souche mésenchymateuse dérivée du sang du cordon ombilical, une cellule souche mésenchymateuse dérivée de la moelle osseuse, une cellule souche mésenchymateuse dérivée du placenta ou une cellule souche mésenchymateuse dérivée du tissu adipeux.

7. Procédé d'inhibition de l'accumulation des graisses, le procédé comprenant: la mise en contact d'une cellule souche mésenchymateuse avec des cellules *in vitro,* la cellule souche mésenchymateuse surexprimant PRL-1 comparée à une cellule mère non modifiée génétiquement.
